# EUROPEAN PATENT SPECIFICATION

(11) **EP 3 854 316 B1**
(45) Date of publication and mention of the grant of the patent: **24.04.2024**
(21) Application number: 19862122.9
(22) Date of filing: 17.09.2019
(51) Int. Cl.: A61B 17/06, A61F 2/00, A61L 17/12, A61L 17/06, A61L 31/14, A61L 31/02, A61K 9/00, A61B 17/00

(54) **THREAD EMBEDDING THERAPY ROPE AND THREAD EMBEDDING THERAPY NEEDLE APPARATUS COMPRISING SAME**
GEWINDEEINGEBETTETES THERAPIESEIL UND GEWINDEEINGEBETTETE THERAPIENADELVORRICHTUNG DAMIT
CÂBLE DE THÉRAPIE D'INCORPORATION DE FIL ET APPAREIL À AIGUILLE DE THÉRAPIE D'INCORPORATION DE FIL COMPRENANT CELUI-CI

(30) Priority: 17.09.2018 KR 20180110982
(43) Date of publication of application: 28.07.2021
(73) Proprietor: Labnpeople Co.,Ltd., Yangju-si, Gyeonggi-do 11477 (KR)
(72) Inventor: CHO, Sung Youn, Uijeongbu-Si, Gyeonggi-do 11812 (KR)
(74) Representative: Jacobi, Markus Alexander
(86) International application number: PCT/KR2019/012002
(87) International publication number: WO 2020/060159

(56) References cited:
- EP-A1- 2 106 752
- WO-A1-2009/111802
- WO-A1-2014/061968
- JP-A- 2005 013 733
- JP-A- 2008 062 061
- KR-A- 20160 000 824
- KR-A- 20170 024 479
- KR-A- 20170 115 429
- US-A1- 2003 149 447
- US-A1- 2011 264 138

## Description

### [TECHNICAL FIELD]

The present invention relates to a thread embedding therapy rope and a thread embedding therapy needle apparatus including the same, and more particularly, to a thread embedding therapy rope using a biodegradable metal, and a thread embedding therapy needle apparatus including the same.

### [BACKGROUND ART]

A thread embedding therapy refers to a therapy, as one of the blood meridian burial therapy, for curing diseases by continuously stimulating a blood meridian after embedding foreign matter in the blood meridian. The purpose of the thread embedding therapy is to increase therapeutic effects by physicochemically stimulating the embedding matter as well as prolonging the stimulation time for the blood meridian. There are significantly various types of buried matters. In the past, animal tissues, such as adrenal, pituitary gland and fat of pigs, sheep, chickens, rabbits, or the like and spleen of dogs, drugs, metal rims, magnetic blocks were used, and currently, metal tools and catguts are used. In Korea, chromic surgical thread is mainly used, sutures mainly formed of polydioxanone (PDO), which are proven in safety, have less tissue reaction, and are hydrated and melted after a predetermined time, are widely used.

The thread embedding therapy is divided into two aspects, the lasting acupuncture and the medicinal thread. First, in regard to the lasting acupuncture, various tools have been developed according to the intensity of stimulus and the type of disease since the Nine Acupuncture of <<Esoteric Scripture of the Yellow Emperor>>. In particular, the scheme of lasting acupuncture was created to give continuous stimulation to stubborn and chronic diseases, and «Last and First of Spiritual Pivot of the Esoteric Scripture of the Yellow Emperor» provided the theoretical basis of the lasting acupuncture.

In the <<Tai Ping Imperial Grace Formulary>> published by the Tai Ping Imperial Grace Formulary Bureau of Chinese Song dynasty around AD 982, there is a record of treatment using the thread called a thread embedding therapy or burying therapy in which the treatment was performed on the blood meridian after smearing drugs in the thread. In acupuncture medicine textbooks used in the oriental medicine education, the acupuncture is divided into an acupuncture according to stimulation site, an acupuncture according to stimulation method, and an acupuncture according to a specific theory. The thread embedding therapy corresponds to the new acupuncture needle method belonging to an instant acupuncture according to the stimulation method.

The thread embedding therapy has indicants in the level of curing various diseases such as acute disease and excess syndrome beyond the boundary line of chronic disease and deficiency syndrome, there are over 200 types of curable diseases, and the contents are used in various fields such as internal medicine, surgery, gynecology, pediatrics, dermatology, ophthalmology, otolaryngology, and musculoskeletal diseases.

In modern medicine, the thread embedding therapy is widely applied in a manner of inserting an insert such as a lifting wire as a method for improving skin aging or wrinkles. The specific causes of skin aging such as wrinkles and loss of elasticity have not been clearly identified even in long-term studies. When an insert is inserted under the skin, such as the subcutaneous insertion of a lifting wire, remarkably direct effects comparable to surgical methods may be obtained. The lifting wire not only has the mechanical effect of pulling the skin under the skin, but also obtains effects such as rejuvenating skin elasticity and spreading wrinkles after cell tissues around the inserted lifting wire are healed and regenerated to promote collagen production in the dermal layer.

Recently, a method is applied in which a suture formed of a biodegradable material is used and the suture in vivo is decomposed after the suture inserted in the dermal layer has fulfilled its role.

Polydioxanone (PDO) is the most commonly and commercially used material for the suture. Recently, the use of poly-L-lactic acid (PLLA) is also increasing in an aspect of properties for allowing the suture to be decomposed in the body for a longer period and human-friendly properties. In addition, based on the decomposition rate, mechanical strength and ductility, polylactic acid (PLA), polyglycolic acid (PGA), polycarporactone (PCL), glycolide-lactide copolymer (PGLA), and a copolymer thereof are applicable for the thread embedding therapy sutures as biodegradable materials. However, since the biodegradable polymeric materials are basically weak in strength, there is a high risk of unwanted breaks occurring during the procedure. In addition, since the biocompatibility is relatively low compared to some certified metal materials, biological rejection reactions such as pain and inflammation trigger may also occur.

As a technology to suggest solutions to some of the above problems, Korean Patent Registration No. 10-1641299 discloses that metal ions or metal particles are deposited on a surface of a biodegradable cog embedding thread to form a metal-coated thin film so as to prevent direct contact between body fluids and the cog embedding thread. However, the deposited metal is limited to gold, silver, and titanium known as having high biocompatibility, so it fails to suggest a clear solution for the decomposition of the metal component in the body. In addition, because the metal is configured to be deposited on a polymer suture in the form of a thin film, it fails to suggest a solution for the reduction in strength of polymer materials, which is a major problem in the related art. In addition, when the surface of the biologically compatible cog embedding thread is deposited with metal ions or metal particles, the cog embedding thread fails to actually function as a medicinal thread. WO 2014/061968 discloses an insertion member with protrusions having a certain shape on the surface, which is injected into the soft tissue of a living body. The insertion member comprises a body including a biodegradable polymer.

Meanwhile, the inventors have conducted the study on the biocompatibility and the strength maintenance of biodegradable Mg-Ca-Zn-based alloy bone implant materials (Cho SY, Chae S-W, Choi KW, Seok HK, Kim YC, Jung JY, Yang SJ, Kwon GJ, Kim JT, Assad M. 2012. Biocompatibility and strength retention of biodegradable Mg-Ca-Zn alloy bone implants. J Biomed Mater Res Part B 2012:00B:000-000). Specifically, the biocompatibility and the strength retention of the Mg-Ca-Zn alloy bone screw were evaluated. As a result, in histopathological analysis using the rabbit transplant model, the Mg-Ca-Zn alloy indicated that no inflammatory cells did not exist at all or rarely existed in the tissue around the implant. In addition, hematology and serum biochemical tests proved that the implant was in the range of acceptable criteria before and after the transplantation, and it has been proved that the release of metallic elements from the implant has no significant effect on normal tissue levels. In this regard, it has been confirmed that the Mg-Ca-Zn-based alloy is an excellent alternative to orthopedic biodegradable polymers.

The present inventors have confirmed the excellent biocompatibility of the Mg-Ca-Zn-based alloy and have attempted to apply the biocompatibility to various fields. As an example, the inventors have tried to use the alloy as a microcarrier. In this regard, the inventors have previously filed an application for the microcarrier including Mg or Zn alone, or Mg or Zn-based metal such as Mg-Zn-Ca-based alloy (Korean Unexamined Patent Publication No. 2017-0115449). For these microcarrier, a human application test was requested to P&K Skin Clinical Research Center and conducted. Specifically, wrinkles around the eyes, skin elasticity, dermal density and skin thickness were measured, the stability was evaluated, and the efficacy and effects were confirmed.

### [DISCLOSURE]

### [TECHNICAL PROBLEM]

The present invention provides a thread embedding therapy rope by using a biodegradable metal including Mg or Zn metal or alloys thereof and having the biocompatibility verified from the research of the inventors, so as to have no side effects on biological tissue when applied to the human body through the thread embedding therapy, strengthen pulling force on the tissue, extend a lifespan compared to a conventional embedded thread of a biodegradable polymer, increase the drug loading capability of the embedded thread itself so as to allow for usefulness as a drug carrier, and deliver a drug through a simple method.

In addition, the present invention provides a thread embedding therapy needle apparatus including the above thread embedding therapy rope.

### [TECHNICAL SOLUTION]

The present invention provides a thread embedding therapy rope inclu ding a linear core including a biodegradable polymer; and
a metal wire spirally wound on an outer circumferential surface of the li near core, wherein
the metal wire includes at least one biodegradable metal selected from magnesium or zinc only or a mixture thereof and an alloy formed of magnesi um or zinc as a main component.

More specifically, according to the present invention, the biodegradabl e metal may be represented by the following Formula 1.

[Formula 1] MgₐZn_{b}X_{c}

In Formula 1, a, b and c are weight percent of each component, a + b + c = 100 wt%, a or b is the biggest in the ranges 0≤a≤100, 0≤b≤100, 0≤c≤3 0, and X is a metal other than magnesium or zinc.

In the thread embedding therapy rope according to one exemplary em bodiment, the metal wire may have a protrusion portion including a plurality o f protrusions irregularly or regularly arranged on at least one surface thereof.

In the thread embedding therapy rope according to one exemplary em bodiment, the metal wire may have a protrusion portion in which protrusions having a triangular section are arranged continuously on one surface thereof.

The thread embedding therapy rope according to one exemplary emb odiment may have a shape of a cog embedding thread.

In the metal wire of the thread embedding therapy rope according to o ne exemplary embodiment, a, b and c are weight percent of each component in Formula 1, a + b + c = 100 wt%, i) 90≤a≤100, 0≤b≤10, 0≤c≤10 or ii) 0≤a≤ 10, 90≤b≤100, 0≤c≤10, and X may include at least one biodegradable metal selected from the group consisting of Ca, Fe, Mn, Si, Na, Zr, Ce, Ag, and P.

In an exemplary embodiment, the metal wire may be Mg having a purit y of 95% or more.

In an aspect of the sustained release and the persistency as a support, the metal wire may be Zn having a purity of 95% or more.

In an exemplary embodiment, the linear core may include at least one biodegradable polymer selected from polydioxanone (PDO), polylactic acid ( PLA), polyglycolic acid (PGA), polycaprolactone (PCL), and copolymer there of.

Another embodiment of the present invention provides a thread embedding therapy needle apparatus including the thread embedding therapy rope according to the embodiments.

### [ADVANTAGEOUS EFFECTS]

The thread embedding therapy rope of the present invention has a shape in which a metal wire including a biodegradable metal or alloy spirally encompasses a linear core including a biodegradable polymer, so that the stiffness of the metal is effectively lowered to have flexible mechanical properties when the biodegradable metal or alloy is applied to the thread embedding therapy, and thus the feeling of foreign matter due to the metal can be minimized, the traction of the rope with respect to tissue can be improved, the mechanism of a specific biodegradable metal or alloy in the body can reduce a contact area between the polymeric linear core and the tissue to reduce the amount of hydrolysis to extend a lifespan of the rope, a drug is efficiently impregnated in the linear core and a space formed by winding the metal wire on the linear core so as to allow for usefulness as a drug carrier, and provide an administration scheme for delivering drugs through a simple way called thread embedding therapy. Further, due to the mechanism of the biodegradable metal or alloy in the body, an additional effect capable of expressing a swelling effect, a so-called filler effect, can be obtained. The biodegradable polymer have a problem that the strength is deteriorated by the sectional area and the concentration of stress due to physical processing. However, according to the present invention, since the linear core itself including the biodegradable polymer is not physically processed, the mechanical properties can be prevented from being deteriorated.

### [ DESCRIPTION OF DRAWINGS]

FIG. 1 shows one embodiment of a thread embedding therapy rope of the present invention, and illustrates one example of a structure in which an outer circumferential surface of a linear core is spirally wrapped with a metal wire having a smooth surface.
FIG. 2 shows one embodiment of a thread embedding therapy rope of the present invention, and illustrates one example of a structure in which an outer circumferential surface of a linear core is spirally wrapped with a metal wire having one surface with a protrusion portion. FIG. 3 is a longitudinal sectional view of the rope according to FIG. 2.
FIG. 4 shows one example of a needle apparatus into which a thread embedding therapy rope of the present invention is inserted.
FIG. 5 is a graph showing the results of measuring the decomposition rate of a sample for each element of a biodegradable metal contained in a metal wire constituting the thread embedding therapy rope of the present invention.
FIG. 6 is a photograph showing the process of implanting the thread embedding therapy rope of the present invention into a transdermal site of a rat.
FIGS. 7 to 8 are images observed with Micro-CT after inserting the thread embedding therapy rope of the present invention into the transdermal site of the rat. FIG. 7 is an image immediately after the insertion, and FIG. 8 is an image in 1 week after the insertion.

### [ BEST MODE]

Hereinafter, the present invention will be described in more detail with reference to the drawings.

As described above, the inventors have studied the biocompatibility and efficacy effects of each applicable use in connection with magnesium or zinc metal alone or alloys containing the same and disclosed the results in the form of a paper or patent application.

The present invention is obtained as a result of continuous researches based on the above studies, and the present invention is disclosed as a result of various efforts for applying the corresponding biodegradable metal as a drug delivery system through the thread embedding therapy.

In order to apply the biodegradable metal to the thread embedding therapy according to the present invention, a thread embedding therapy rope includes a linear core including a biodegradable polymer; and a metal wire spirally wound on an outer circumferential surface of the linear core, wherein the metal wire includes at least one biodegradable metal selected from magnesium or zinc only or a mixture thereof, and an alloy formed of magnesium or zinc as a main component.

In the thread embedding therapy rope according to the present invention, a biodegradable metal, especially the biodegradable metal including magnesium or zinc is effective for the metal wire as a whole, and the present invention is not limited thereto. As an example, the metal wire may include a biodegradable metal represented by the following Formula 1.

[Formula 1] MgaZnbXc

In Formula 1, a, b and c are weight percent of each component, a + b + c = 100 wt%, a or b is the biggest in the ranges 0≤a≤100, 0≤b≤100, and 0≤ c≤30, and X may be a metal other than magnesium or zinc. Herein, X may be a metal known as preventing the inhibition of biodegradability of the alloy, or may be a metal that is easily discharged outside the body or does not cause toxicity during remaining in the human body even when the biodegradability is rare or does not exist.

The biodegradable metal may preferably contain the largest amount of magnesium or zinc. Accordingly, in Formula 1, a, b and c are weight percent of each component, a + b + c = 100 wt%, i) 90≤a≤100, 0≤b≤10 and 0≤c≤10 or ii) 0≤a≤10, 90≤b≤100 and 0≤c≤10, X may be preferably at least one selected from the group consisting of Ca, Fe, Mn, Si, Na, Zr, Ce, Ag, and P.

The biodegradable metal according to the present invention is a metal absorbed and decomposed in tissues to release metal ions and decomposition products into the body when applied for the thread embedding therapy. Magnesium (Mg), calcium (Ca), zinc (Zn) and the like are alkaline earth metal-based biodegradable metals, and have a mechanism of releasing hydrogen gas after reacting with water as represented by the following reaction formulae 1 to 3.

[Reaction Formula 1] Mg + 2H₂O → Mg(OH)₂ + H₂ (gas)

[Reaction Formula 2] Ca + 2H₂O → Ca(OH)₂ + H₂ (gas)

[Reaction Formula 3] Zn + 2H₂O → Zn(OH)₂ + H₂ (gas)

According to the present invention, the biodegradable metal preferably may be manufactured using magnesium (Mg) or zinc (Zn) as a single material, and a biodegradable metal using magnesium as a single material may be preferable in an aspect of having excellent biocompatibility and expressing no toxicity in normal cells or tissues. However, when a function as a long-term support or a continuous and long-term drug-release is taken into consideration, zinc having a slower decomposition rate compared to magnesium may be preferable. It will be understood that the magnesium refers to magnesium having a purity of 95% or more, and the zinc will also be understood as zinc having a purity of 95% or more.

In the thread embedding therapy rope the biodegradable metal material may have a significantly faster decomposition rate when used alone as a long-term support. The above problem may be solved by using a biodegradable polymer as a core wire to mutually extend lifespans.

Meanwhile, the biodegradable metal represented by Formula 1 may be controlled in the decomposition rate thereof by including intermetallic compounds having different potentials. For example, at least two metal phases may form a galvanic circuit and accelerate the decomposition rate.

Specifically, the biodegradable metal may include a biodegradable metal containing a Mg₂Ca phase, a MgZn phase, or a Ca ₂Mg₆Zn₃ phase.

As another way to control the decomposition rate, a surface of the biodegradable metal represented by Formula 1 may be coated with a plasma electrolytic oxidation (PEO), a polymer, or another type of second metal. The second metal may be sodium, magnesium, potassium, iron, nickel, zinc, gallium, selenium, strontium, zirconium, molybdenum, niobium, tantalum, titanium, silicon, silver, gold, manganese, calcium or the like as an example, but the second metal is not limited thereto. The corrosion rate and biocompatibility may be maintained when iron (Fe) is included, especially when chromium (Cr) and nickel (Ni) functioning as stainless are controlled to less than 1% by mass.

The biodegradable metal according to the present invention as described above itself may have antibacterial activity against acne causative bacteria. The inventors of the present invention prepared a biodegradable metal formed of magnesium or zinc as a single material and a biodegradable metal formed by mixing other types of metals such as calcium with the above biodegradable metal into a thin plate shape, and evaluated the antibacterial activity against acne causative bacteria. As a result, it was confirmed that the biodegradable metal itself prepared using magnesium, calcium or zinc as a single material, or the alloy itself formed by mixing the other types of metals with the above metal in a specific range has the antibacterial activity. Accordingly, a cosmetic composition including the metals for relieving and preventing acne has been disclosed (Korean Patent Application No. 10-2018-0078402).

According to the above disclosure, it is expected that the thread embedding therapy rope of the present invention may express the antibacterial activity even against propionibacterium acnes as acne causative bacteria upon treatment according to the thread embedding therapy, so that the acne may be prevented or the activated acne may be relieved.

Meanwhile, the linear core of the thread embedding therapy rope of the present invention includes a biodegradable polymer, and various biodegradable polymers known as applied or applicable to the conventional thread embedding therapy or suture may be used for the biodegradable polymer. As a specific example, the biodegradable polymer may include polydioxanone (PDO), polylactic acid (PLA), polyglycolic acid (PGA), polycaprolactone (PCL), and a copolymer thereof, and the present invention is not limited thereto.

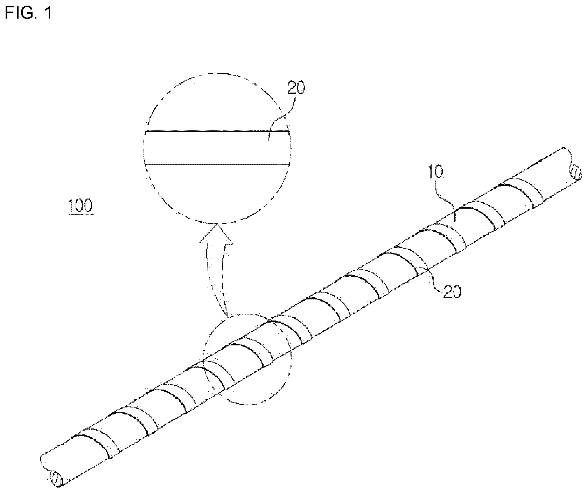

FIG. 1 shows an example of the thread embedding therapy rope of the present invention. The thread embedding therapy rope 100 is configured such that a metal wire 20 including a biodegradable metal or alloy is spirally wrapped an outer circumferential surface of a linear core 10 including a biodegradable polymer.

One of the reasons why the metal wire 20 is arranged to be spirally wrapped around the linear core 10 is to lower the stiffness of the biodegradable metal or alloy. When only the biodegradable metal or alloy having a wire shape is applied for a thread embedding therapy rope, the biosafety may be secured, but the feeling of foreign matter may increase. However, when the metal wire is spirally wrapped around the biodegradable polymeric linear core as described in the present invention, the metal may have more flexible mechanical properties, so that the feeling of foreign matter may be minimized.

The drug impregnation is another reason for the arrangement. In addition to a function as the conventional medicinal thread of the linear core including the biodegradable polymer, that is, a function as a drug carrier, it can be expected that the drug impregnation efficiency may be spatially improved through a microscopic space naturally generated while the biodegradable metal spirally surrounds the linear core.

For example, drugs such as hyaluronic acid are required to be applied in a liquid form so as to be impregnated in a linear core including a polymer. Since hyaluronic acid has extremely low water solubility, the amount of drugs impregnated in the linear core is extremely limited. On the contrary, since the thread embedding therapy rope of the present invention can impregnate the drugs through the microscopic space, so that the actual carrying amount may be increased even when an aqueous solution of hyaluronic acid is used. In addition drugs having low water solubility, such as hyaluronic acid, may be impregnated as a powder or granule itself other than an aqueous solution state. Accordingly, the amount of carried drug can be increased to the substantially required amount.

In addition, it may be preferable in an aspect of improving the traction of the thread embedding therapy rope with respect to the tissue since the spirally wrapped metal wire may provide irregularities on the rope surface.

According to one exemplary embodiment of the present invention, it is preferable in the aspect of more stably securing the microscopic space for improving the drug impregnation efficiency, and in the aspect of more firmly improving the traction for the tissue. The metal wire may have a protrusion portion including a plurality of protrusions irregularly or regularly arranged on at least one surface thereof.

In the descriptions as above and below, the term "a plurality of protrusions irregularly or regularly arranged" will be understood as a term including the protrusions the same or different in size, the protrusions arranged to be continuous or incontinuous, the protrusions the same or different in shape, and combinations thereof.

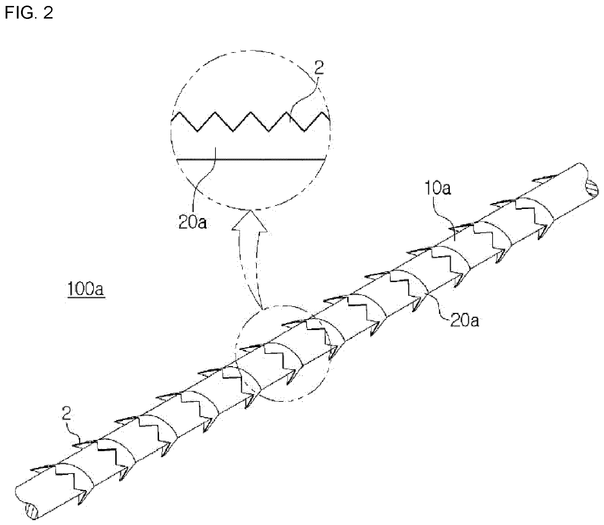

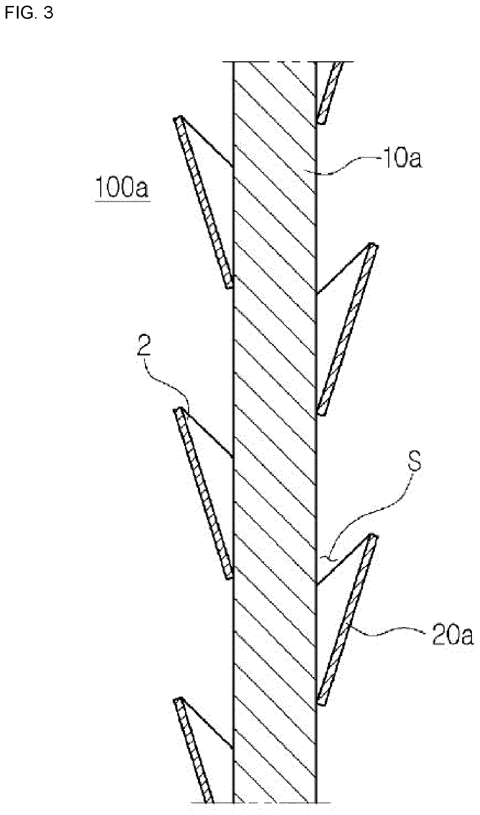

FIGS. 2 and 3 show examples of the thread embedding therapy rope according to one exemplary embodiment. Referring to the above drawings, the metal wire 20a may have a protrusion portion 2 in which protrusions having a triangular section are arranged continuously on one surface thereof.

When the metal wire 20a having the above shape is spirally wound around the linear core 10a, the thread embedding therapy rope 100a has a longitudinal section in the form of a cog embedding thread (shown as FIG. 3), accordingly, a naturally formed microscopic space S has a size or shape capable of stably carrying drugs, and the cog shape enables the traction for the tissue to be strengthened.

The thread embedding therapy rope according to the present invention and shown in FIGS. 1 to 3 is merely illustrative with reference to the technical idea of the present invention, and the present invention is not limited thereto.

The thread embedding therapy rope according to the present invention has a shape in which a metal wire including a biodegradable metal or alloy spirally encompasses a linear core including a biodegradable polymer, so that a contact area between the biodegradable polymeric linear core and the tissue may be reduced. Further, Based on the reaction mechanism largely represented by the reaction formulae 1 to 3 of the biodegradable metal or alloy, the contact area between the biodegradable polymeric linear core and the tissue is reduced even by generation of hydrogen gas or other oxides, so that, ultimately, the remaining life of the rope in the tissue may be increased.

Accordingly, the rope of the present invention may not only function as a useful drug delivery system in various diseases requiring long-term drug administration, but also be advantageous in terms of the effectiveness of the thread embedding therapy functioning as a support in the tissue.

According to the thread embedding therapy, self-recovering materials in the body are induced to gather around the rope of the present invention, so that weakened muscles may be thickened and strengthened. In addition, the rope is recognized as foreign matter in the body to promote continuous biochemical stimulation and tissue recovery action on a site to which the procedure is applied, and the long-lasting physical and chemical stimulations enable changes in structures and functions of a human body, so that stubborn and chronic diseases may be effectively treated. The above thread embedding therapy may be applied to lumbar disc, neck disc, facial paralysis sequela, facial cosmetic treatment, knee arthritis, frozen shoulder, facial asymmetry, partial obesity, or the like so as to serve as a support for muscles and ligaments and improve the blood circulation, so that the effects on pain diseases and cosmetic plastic surgery may be implemented.

Further, when the rope of the present invention is used for the above various thread embedding therapies, it may be preferable in that the drug impregnation efficiency may be maximized and the lifespan of the rope may be expanded, thereby functioning as a drug delivery system effective for the chronic diseases treatment, so that the medicinal effect lasts for a long time with one administration. Thus, side effects caused by frequent and long-term drug administration of chronical disease patients may be reduced, and a local drug administration may be conducted in a simple way.

In addition, the traction within the tissue is maximized so that the rope may serve as a support for muscles and ligaments and may be useful as a lifting wire capable of effectively improving a face that is sagging and has many wrinkles.

Meanwhile, based on the reaction mechanism of the biodegradable metal or alloy largely divided by the above reaction formulae 1 to 3, hydrogen gas may be generated in the tissue to implement a swelling effect. Accordingly, it can be expected that the rope according to the present invention may be useful as a solid filler through the thread embedding therapy. In regard to the filler, recently, liquid fillers such as hyaluronic acid are commonly used. There are many cases of rejection reaction against hyaluronic acid injected in large quantities. In addition, when the injected filler is required to be removed due to the biological rejection reaction or the like, a decomposition enzyme such as hyaluronase is used to perform the removal procedure, but it is difficult to completely remove the injected filler. Accordingly, as there are needs for the easy removal, the number of surgeons who want easily removable solid fillers is increasing. In this case, the filler injection, ease of inserting procedure, ease of product forming, and biocompatibility, and the like are required as major characteristics. In addition, needs for solid fillers that add advantageous effects for cell activation in tissues are increasing. The rope according to the present invention may be useful as a solid filler that may satisfies the above needs.

Meanwhile, in the thread embedding therapy rope according to the present invention, the thickness or the like of the linear core is not limited as long as it is acceptable for a biodegradable polymeric filament of the thread embedding therapy. The metal wire including the biodegradable metal introduced according to the present invention may preferably have a filament thickness comparable to the thickness of the biodegradable polymeric filament, based on the feeling of foreign matter in the tissue and the ease of spirally winding. In view of common ranges, the linear core and the metal wire may be 18 gauge to 30 gauge, but the present invention is not limited thereto.

When the rope of the present invention having the above various effects is applied to the thread embedding therapy, the rope may be provided in the form of various needle apparatuses according to the usual thread embedding therapy. The thread embedding therapy refers to a therapy in which the above-described rope is inserted into a needle that is variously designed, the needle is injected into a site to be treated, and only the rope remains in the body to continuously treat diseases. Various types of needles may be coupled to the rope.

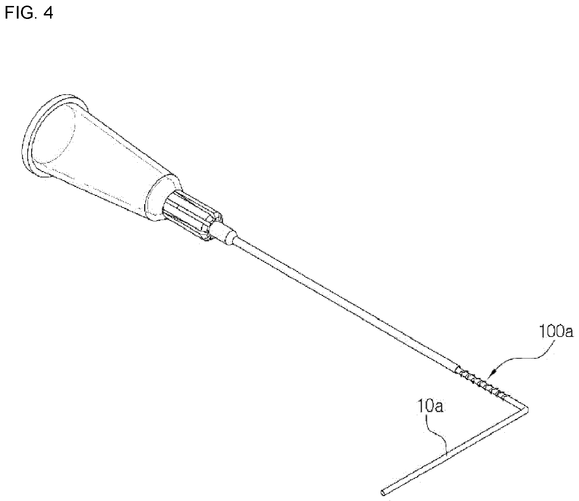

FIG. 4 shows one example of a needle apparatus 200 into which the rope 100a of the present invention is inserted, but the present invention is not limited thereto.

In FIG. 4, it is preferable to insert the needle in a state in which only the portion of the linear core 10a is exposed while a portion including the metal wire is included in the needle so as to prevent resistance or scratch when the rope 100a is inserted into the tissue. However, it should be understood that the drawing illustrates the linear core having an exposed portion of the rope 100a of the present invention to show the rope 100a is inserted into the needle.

### [Mode for Invention]

Hereinafter, the present invention will be described in detail based on examples, and the present invention is not limited by the examples.

### <Reference Example>

The evaluation on the stability of skin against a biodegradable metal contained in the metal wire constituting the rope of the present invention has already been clarified from the research paper or the patent application by the inventors as described in the Background Art.

Accordingly, the biodegradable metal wire of the present invention may be introduced into the skin without irritation and toxicity in normal cells or tissues.

The following examples show examples of the decomposition properties according to the composition of metal elements for the biodegradable metal contained in the metal wire constituting the rope of the present invention. These are examples to show that the degradation rate in vivo is controllable in various ways when the biodegradable metal is wound on the linear core known as the biodegradable polymer.

A metal sheet (having 60 mm and 1.5 mm in left and right widths after casted in a high-frequency melting furnace and extruded at a rate of 0.1 mm/s) was prepared with the composition (% by weight) of Table 1 below.

**[TABLE 1]**

| Sample (Example) | Mg | Ca | Zn |
|---|---|---|---|
| 1 | 99.99* | - | - |
| 2 | 98.35 | 0.05 | 1.60 |
| 3 | 98.95 | 0.05 | 1.00 |
| 4 | 98.9 | 0.10 | 1.00 |
| 5 | 98.85 | 0.15 | 1.00 |
| 6 | 96.9 | 0.10 | 3.00 |
| 7 | 96.85 | 0.15 | 3.00 |
| 8 | 0.00 | 0.00 | 99.99* |
| 9 | 10.00 | 0.00 | 90.00 |

| | | | |
|---|---|---|---|
| * Pure metal containing inevitable impurities generated during the preparation | | | |

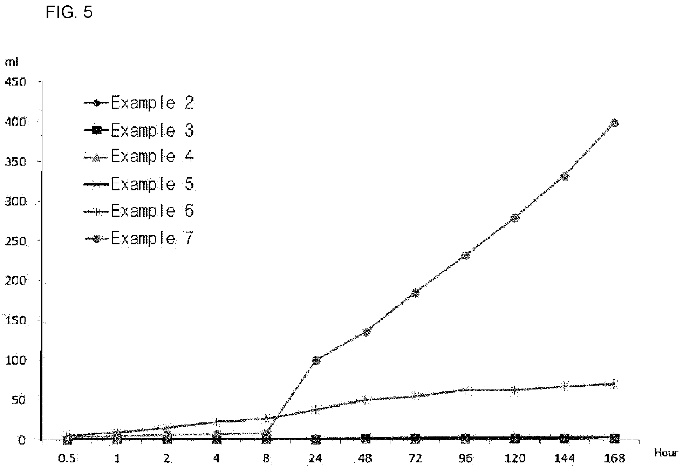

After metal sheets manufactured in Samples 2 to 7 were immersed in a eudiometer containing a biomimetic solution having the composition of Table 2, the decomposition rate is evaluated by the amount of hydrogen generated according to the immersion time, and the results are shown in FIG. 5.

**[TABLE 2]**

| Component | Molar concentration [mM/L] | Mass [g] |
|---|---|---|
| CaCl₂·2H₂O | 1.26 | 0.185 |
| KCl | 5.37 | 0.400 |
| KH₂PO₄ | 0.44 | 0.060 |
| MgSO₄·H₂O | 0.81 | 0.200 |
| NaCl | 136.89 | 8.000 |
| Na₂HO₄·2H₂O | 0.34 | 0.060 |
| NaHCO₃ | 4.17 | 0.350 |
| D-Glucose | 5.55 | 1.000 |

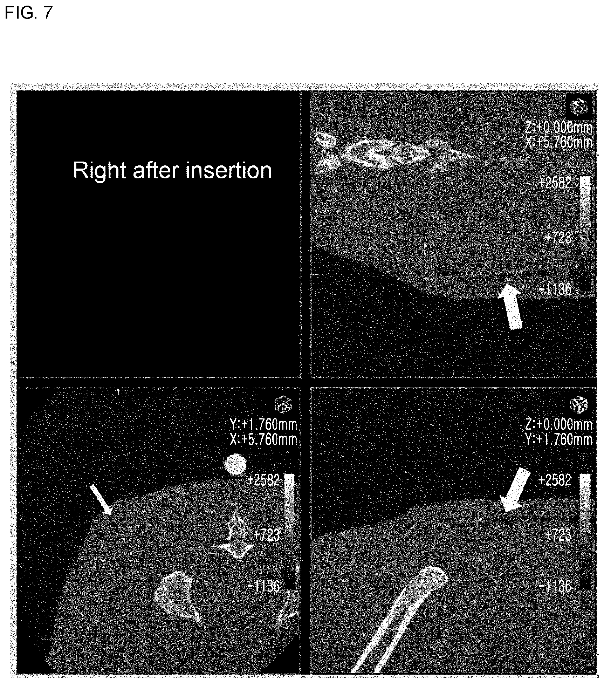

As shown in FIG. 7, the metal sheets of Samples 2 to 5 (Examples 2 to 5), in which a Mg 2Ca phase was not generated, shows the amount of hydrogen gas generated due to stable decomposition, but the amount of hydrogen gas generated in the metal sheets of Samples 6 and 7, in which the Mg 2Ca phase was generated, is significantly higher compared to other examples. Thus, it is confirmed that the decomposition rate is increased. Accordingly, it is confirmed that the decomposition rate of the biodegradable metal can be controlled by controlling the composition.

### <Example 1>

A needle apparatus for the thread embedding therapy as shown in FIG. 4 is manufactured according to the specification shown in Table 3 below.

**[Table 3]**

| Shape of metal wire | Linear core component | Needle (W Type) | | Linear core (suture) | | |
|---|---|---|---|---|---|---|
| | | Gauge | Length (mm) | USP standard | Overall length (mm) | Length of portion wrapped by metal wire of the overall length (mm) |
| Shape of metal wire | Polydioxanone. Polycaprolactone | 18G | 38 | 0 | 100 | 30 |
| | | | 50 | | 110 | 40 |
| of FIG. 1 or 2 (For example, metal wire formed of magnesium having a purity of 95%; Mg-Zn-Ca alloy wire; Or Mg-Zn-Ca-Mn alloy wire) | or polyglycolide-lactide copolymer | | 60 | | 120 | 50 |
| | | | 100 | | 170 | 90 |
| | | 19G | 38 | 2-0 | 100 | 30 |
| | | | 50 | | 110 | 40 |
| | | | 60 | | 120 | 50 |
| | | | 100 | | 170 | 90 |
| | | 21G | 38 | 3-0 | 100 | 30 |
| | | | 50 | | 110 | 40 |
| | | | 60 | | 120 | 50 |

According to the specification shown in Table 3, it can be seen that the length of the portion wrapped by the metal wire among the total length of the linear core is adjusted in consideration of the length of the needle. In other words, it can be confirmed that the length of the portion wrapped by the metal wire is required to be prevented from exceeding the length of the needle, so that the portion wrapped by the metal wire is completely included inside the needle. It is preferable to insert the needle in a state in which only the portion of the linear core is exposed while a portion including the metal wire is included in the needle, in the aspect of preventing resistance or scratch when the rope for the thread embedding therapy according to the present invention is inserted into the tissue.

In the above specification, the shape has been described in relation to the metal wire. The metal wire is applied in the shape of spirally winding the linear core, in which the width, thickness, and length of the metal wire may be substantially variously adjusted based on the suture standard and the gauge and length of the needle.

### <Experimental Example>

In order to confirm that the thread embedding therapy rope of the present invention generates hydrogen gas in the tissue, and that the contact area between the polymeric linear core and the tissue is reduced due to the generation of hydrogen gas, thereby expanding the lifespan of the rope, an experiment was conducted to a rat as a subject in which the thread embedding therapy rope was inserted into a transdermal layer and an observation was performed through Micro-CT.

Specifically, the thread embedding therapy needle apparatus was manufactured as shown in FIG. 4 by using polydioxanone as the linear core and preparing a thread embedding therapy rope (the length of the portion wrapped by the metal wire is 50 mm out of 70 mm in total length) in which a metal wire formed of magnesium having a purity of 95% (0.07 mm in thickness and 0.7 mm in width) wraps the linear core in a shape as shown in FIG. 1.

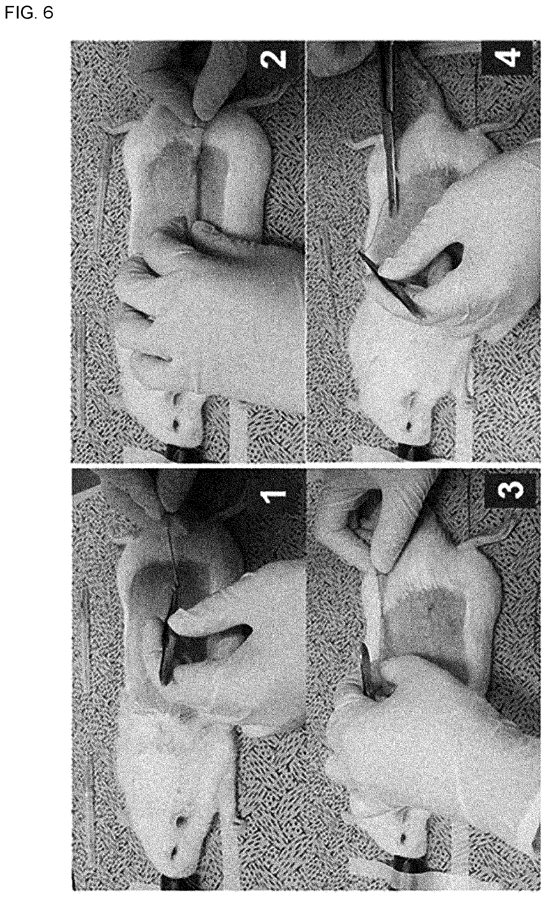

The needle was inserted into the transdermal layer of the rat according to the sequence shown in FIG. 6.

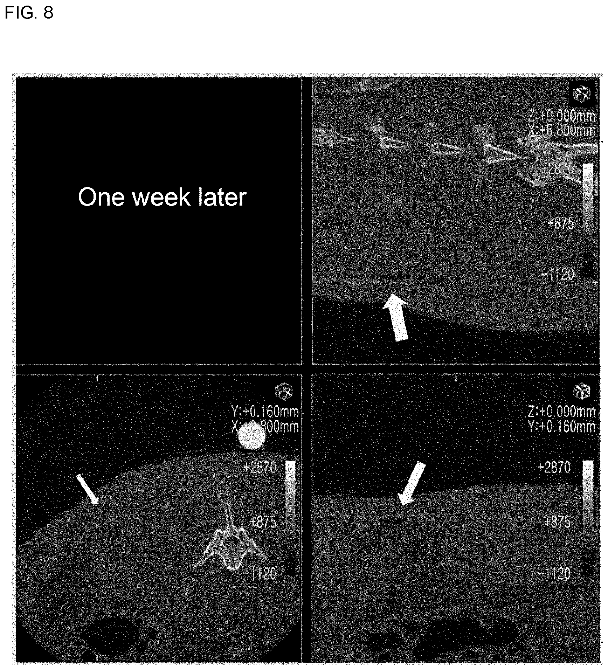

As a result of observation with Micro-CT (80 µm in resolution and 40 mm in FOV) on the rat after completion of the procedure, FIGS. 7 and 8 show the results that air pockets were irregularly generated immediately after insertion.

FIG. 7 is a Micro-CT image immediately after the thread embedding therapy rope is inserted, and FIG. 8 is a Micro-CT image in 1 week after the insertion.

The portions indicated by arrows in FIGS. 7 and 8 refer to portions in which the metal wire wraps the linear core. Only the linear core cannot be displayed on the Micro-CT.

In the portion indicated by the arrows in FIGS. 7 and 8, black spots, which are present around the rope, correspond to the air pockets caused by the generation of hydrogen gas as a decomposition product of magnesium.

The above results also show that an additional effect capable of expressing a swelling effect, a so-called filler effect, may be obtained due to the mechanism of the biodegradable metal or alloy in the body.

### [Industrial Applicability]

The thread embedding therapy rope and the thread embedding therapy needle apparatus including the thread embedding therapy rope according to the present invention may be useful as an instrument so as to have no side effects on biological tissue when applied to the human body by means of thread embedding therapy, strengthen pulling force on the tissue, extend a lifespan compared to a conventional embedded thread of a biodegradable polymer, increase the drug loading capability of the embedded thread itself so as to allow for usefulness as a drug carrier, and deliver a drug through a simple method.

## Claims

1. A thread embedding therapy rope (100, 100a), which is a suture for thread-embedding therapy, comprising: a linear core (10, 10a) including a biodegradable polymer; and a metal wire (20, 20a) spirally wound on an outer circumferential surface of the linear core (10, 10a), wherein the metal wire (20, 20a) includes at least one biodegradable metal selected from magnesium or zinc only or a mixture thereof, and an alloy formed of magnesium or zinc as a main component.

2. The thread embedding therapy rope (100, 100a) of claim 1, wherein the biodegradable metal is represented by Formula 1.
[Formula 1] MgaZnbXc
In Formula 1, a, b and c are weight percent of each component, a + b + c = 100 wt%, a or b is the biggest in ranges of 0≤a≤100, 0≤b≤100, and 0 ≤c≤30, and X is a metal other than magnesium or zinc.

3. The thread embedding therapy rope (100a) of claim 1, wherein the metal wire (20a) has a protrusion portion (2) including a plurality of protrusions irregularly or regularly arranged on at least one surface thereof.

4. The thread embedding therapy rope (100a) of claim 1, wherein the metal wire (20a) has a protrusion portion (2) in which protrusions having a triangular section are arranged continuously on one surface thereof.

5. The thread embedding therapy rope (100a) of claim 4, wherein the thread embedding therapy rope (100a) has a shape of a cog embedding thread.

6. The thread embedding therapy rope (100, 100a) of claim 2, wherein, in the metal wire (20, 20a) with Formula 1, a, b and c are weight percent of each component, a + b + c = 100 wt%, i) 90≤a≤100, 0≤b≤10 and 0≤c≤10, or ii) 0 ≤ a ≤ 10, 90 ≤ b ≤ 100, 0 ≤ c≤ 10, and X includes at least one biodegradable metal selected from the group consisting of Ca, Fe, Mn, Si, Na, Zr, Ce, Ag, and P.

7. The thread embedding therapy rope (100, 100a) of claim 1, wherein the metal wire (20, 20a) includes Mg having a purity of 95% or more.

8. The thread embedding therapy rope (100, 100a) of claim 1, wherein the metal wire (20, 20a) includes Zn having a purity of 95% or more.

9. The thread embedding therapy rope (100, 100a) of claim 1, wherein the linear core (10, 10a) includes at least one biodegradable polymer selected from polydioxanone, polylactic acid, poly-L-lactic acid, polyglycolic acid, polycaprolactone, and a copolymer thereof.

10. A thread embedding therapy needle apparatus including a thread embedding therapy rope (100, 100a) of any one of claims 1 to 9.

## Patentansprüche

1. Fadeneinbettendes Therapieseil (100, 100a), das ein Nahtmaterial für die Fadeneinbettungstherapie ist, umfassend: einen linearen Kern (10, 10a), der ein biologisch abbaubares Polymer enthält; und einen Metalldraht (20, 20a), der spiralförmig um eine äußere Umfangsfläche des linearen Kerns (10, 10a) gewickelt ist, wobei der Metalldraht (20, 20a) mindestens ein biologisch abbaubares Metall als Hauptkomponente enthält, das nur aus Magnesium oder Zink oder einem Gemisch davon und einer aus Magnesium oder Zink gebildeten Legierung ausgewählt ist.

2. Fadeneinbettendes Therapieseil (100, 100a) gemäß Anspruch 1, wobei das biologisch abbaubare Metall durch Formel 1 dargestellt ist:
[Formel 1] MgₐZn_{b}X_{c}
in Formel 1 a, b und c Gewichtsprozent jeder Komponente sind, a + b + c = 100 Gew.-%, a oder b das Größte in den Bereichen 0 ≤ a ≤100, 0 ≤ b ≤ 100, und 0 ≤ c ≤ 30 ist, und X ein Metall ist, das nicht Magnesium oder Zink ist.

3. Fadeneinbettendes Therapieseil (100a) gemäß Anspruch 1, wobei der Metalldraht (20a) einen Vorsprungsteil (2) aufweist, der eine Vielzahl von Vorsprüngen enthält, die unregelmäßig oder regelmäßig auf mindestens einer seiner Oberflächen angeordnet sind.

4. Fadeneinbettendes Therapieseil (100a) nach Anspruch 1, wobei der Metalldraht (20a) einen Vorsprungsteil (2) aufweist, in dem Vorsprünge mit einem dreieckigen Querschnitt kontinuierlich auf einer Oberfläche davon angeordnet sind.

5. Fadeneinbettendes Therapieseil (100a) nach Anspruch 4, wobei das Fadeneinbettende Therapieseil (100a) eine Form eines gezahnten einbettenden Fadens hat.

6. Fadeneinbettendes Therapieseil (100, 100a) nach Anspruch 2, wobei in dem Metalldraht (20, 20a) mit Formel 1, a, b und c Gewichtsprozente jeder Komponente sind, a + b + c = 100 Gew.-%, i) 90 ≤ a ≤ 100, 0 ≤ b ≤ 10 und 0 ≤ c ≤ 10, oder ii) 0 ≤ a ≤ 10, 90 ≤ b ≤ 100, 0 ≤ c ≤ 10, und X mindestens ein biologisch abbaubares Metall, ausgewählt aus der Gruppe bestehend aus Ca, Fe, Mn, Si, Na, Zr, Ce, Ag und P, umfasst.

7. Fadeneinbettendes Therapieseil (100, 100a) nach Anspruch 1, wobei der Metalldraht (20, 20a) Mg mit einer Reinheit von 95% oder mehr enthält.

8. Fadeneinbettendes Therapieseil (100, 100a) nach Anspruch 1, wobei der Metalldraht (20, 20a) Zn mit einer Reinheit von 95% oder mehr enthält.

9. Fadeneinbettendes Therapieseil (100, 100a) nach Anspruch 1, wobei der lineare Kern (10, 10a) mindestens ein biologisch abbaubares Polymer ausgewählt aus Polydioxanon, Polymilchsäure, Poly-L-Milchsäure, Polyglykolsäure, Polycaprolacton und einem Copolymer davon enthält.

10. Fadeneinbettungstherapie-Nadelgerät umfassend ein fadeneinbettendes Therapieseil (100, 100a) nach einem der Ansprüche 1 bis 9.

## Revendications

1. Corde de thérapie d'incorporation de fil (100, 100a), qui est une suture pour une thérapie d'incorporation de fil, comprenant : une âme linéaire (10, 10a) comportant un polymère biodégradable ; et un fil métallique (20, 20a) enroulé en spirale sur une surface circonférentielle externe de l'âme linéaire (10, 10a), dans laquelle le fil métallique (20, 20a) comprend au moins un métal biodégradable choisi parmi le magnésium ou le zinc uniquement ou un mélange de ceux-ci, et un alliage formé de magnésium ou de zinc en tant que composant principal.

2. Corde de thérapie d'incorporation de fil (100, 100a) selon la revendication 1, selon laquelle le métal biodégradable est représenté par la formule 1.
[Formule 1] MgaZnbXc
Dans la formule 1, a, b et c sont des pourcentages en poids de chaque composant, a + b + c = 100 % en poids, a ou b est le plus grand dans les plages de 0 ≤ a ≤ 100, 0 ≤ b ≤ 100, et 0 ≤ c ≤ 30, et X est un métal autre que le magnésium ou le zinc.

3. Corde de thérapie d'incorporation de fil (100a) selon la revendication 1, dans laquelle le fil métallique (20a) présente une partie de protubérances (2) comportant une pluralité de protubérances agencées de manière irrégulière ou régulière sur au moins une surface de celle-ci.

4. Corde de thérapie d'incorporation de fil (100a) selon la revendication 1, dans laquelle le fil métallique (20a) présente une partie de protubérances (2) dans laquelle des protubérances présentant une section triangulaire sont agencées de manière continue sur une surface de celle-ci.

5. Corde de thérapie d'incorporation de fil (100a) selon la revendication 4, dans laquelle la corde de thérapie d'incorporation de fil (100a) a une forme de fil à incorporation de dents.

6. Corde de thérapie d'incorporation de fil (100, 100a) selon la revendication 2, dans laquelle, dans le fil métallique (20, 20a) avec la formule 1, a, b et c sont des pourcentages en poids de chaque composant, a + b + c = 100 % en poids, i) 90 ≤ a ≤ 100, 0 ≤ b ≤ 10 et 0 ≤ c ≤ 10, ou ii) 0 ≤ a ≤ 10, 90 ≤ b ≤ 100, 0 ≤ c ≤ 10, et X comporte au moins un métal biodégradable choisi dans le groupe constitué par Ca, Fe, Mn, Si, Na, Zr, Ce, Ag et P.

7. Corde de thérapie d'incorporation de fil (100, 100a) selon la revendication 1, dans laquelle le fil métallique (20, 20a) comporte Mg ayant une pureté égale ou supérieure à 95 %.

8. Corde de thérapie d'incorporation de fil (100, 100a) selon la revendication 1, dans laquelle le fil métallique (20, 20a) comporte Zn ayant une pureté égale ou supérieure à 95 %.

9. Corde de thérapie d'incorporation de fil (100, 100a) selon la revendication 1, dans laquelle l'âme linéaire (10, 10a) comporte au moins un polymère biodégradable choisi parmi la polydioxanone, l'acide polylactique, l'acide poly-L-lactique, l'acide polyglycolique, la polycaprolactone, et un copolymère de ceux-ci.

10. Appareil à aiguille de thérapie d'incorporation de fil comportant une corde de thérapie d'incorporation de fil (100, 100a) selon l'une quelconque des revendications 1 à 9.
